# EUROPEAN PATENT APPLICATION

(11) **EP 4 560 023 A1**
(43) Date of publication of application: **28.05.2025**
(21) Application number: 23307043.2
(22) Date of filing: 24.11.2023
(51) Int. Cl.: C12P 13/04, C07C 227/40, C07C 227/42, C12P 17/12

(54) **METHOD OF PURIFICATION OF ECTOINE AND COMPOSITION THEREOF**

(71) Applicant: Metabolic Explorer, 63360 Saint Beauzire (FR)
(72) Inventor: COLOMB, Cédric, 63720 Ennezat (FR); RIFFLART, Sébastien, 63960 Veyre-Monton (FR); CELLIER, Clément, 51420 Witry-Les-Reims (FR)
(74) Representative: Regimbeau

(57) **Abstract**

The present invention relates to a process for the extraction and purification of ectoine from a fermentation broth, comprising at least the successive steps of:
a) Passing said fermentation broth through a cation exchange resin able to capture ectoine, and eluting ectoine, to obtain an ectoine aqueous solution;
b) Concentrating said ectoine aqueous solution and then drying it, to obtain raw crystals of ectoine;
c) Dissolving said raw crystals of ectoine into hot methanol;
d) Hot filtrating said methanol-ectoine mixture;
e) Crystallizing ectoine by cooling of the filtered methanol-ectoine mixture, and recovering high purity ectoine crystals.

## Description

### Field of the invention

The present invention relates to a method of purification of ectoine from a fermentation broth, and to the ectoine composition such as obtained.

### Background of the invention

Ectoine, also known as 1,4,5,6-tetrahydro-2-methyl-4-pyrimidinecarboxylic acid or (S)-2-methyl-3,4,5,6-tetrahydropyrimidine-4-carboxylic acid, is a polar, soluble, uncharged cyclic amino acid derivative. Its CAS number is 96702-02-03. First discovered in the halophilic microorganism *Halorhodospira halochloris,* ectoine functions as an osmolyte, protecting membranes, enzymes, nucleic acids, etc. from high osmotic stress/dehydration. It has also been found to protect cells against other stresses including UV radiation, heating, freezing, and chemical agents. In view of its protective effect, ectoine has a wide variety of commercial applications in cosmetics, oral and skin care products, and as a cryoprotectant or protein stabilizer (Goraj et al., 2019). Ectoine is also of interest in the pharmaceutical industry in view of its potential therapeutic applications, in particular its use in the treatment of inflammation or neurodegenerative disease. Production of ectoine is estimated at approximately 15 tons per year worldwide, with ectoine representing a valuable commodity, retailing at an estimated $1000/kg (Strong et al., 2016).

In halophilic microorganisms, ectoine is synthesized from the L-aspartate-β-semialdehyde (ASA) precursor in three steps with the enzymes EctA, EctB, and EctC. The diaminobutyric acid transaminase (EctB, also referred to as a diaminobutyric acid aminotransferase) initially converts ASA to L-2,4-diaminobutyric acid (DABA). DABA is then converted to Ny-acetyl-L-2,4-diaminobutyric acid by the DABA acetyltransferase (EctA). Finally, ectoine synthase (EctC) converts Ny-acetyl-L-2,4-diaminobutyric acid to ectoine. Genes encoding these enzymes are organized in either *ectABC* or *ectABC-ask* operons with ask coding for an aspartokinase and are generally expressed in response to high salt conditions and temperature extremes.

While ectoine may be chemically synthesized (see, e.g., Goraj et al., 2019, JPH0331265, WO 2010/006792), this is not competitive with microbial processes due to the high cost of precursors, such as DABA, reaction complexity and low stereo-specificity of synthesized ectoine. In contrast, processes using microorganisms having the appropriate metabolic pathway represent a sustainable way to produce ectoine at a lower cost. Indeed, not only are such processes generally more environmentally friendly, enzyme stereoselectivity leads to the production of L-ectoine only.

Microbial production processes are mainly based on so-called "bacterial milking" in which halophilic bacteria, such as *Halomonas elongata* or *Chromohalobacter salexigens,* are initially cultivated in conditions of high salinity (e.g., 10-20% NaCl), inducing ectoine production, and then subjected to a hypoosmotic shock (e.g., 2% NaCl), causing cytoplasmic ectoine to be released into the culture medium. Ectoine titers obtained based on this technique have been reported as ranging from 6.04 g/L to 32.9 g/L (Chen et al., 2017; Fallet et al., 2010). However, high salt levels inevitably cause equipment corrosion and increase the complexity of downstream processing as the recovered product must be desalted, which in turn increases cost. Alternating between high and low salt conditions furthermore results in discontinuous production of ectoine. Yield may also be reduced in such bacteria due to their ability to catabolize ectoine, reusing it as a source of carbon.

In particular, in naturally-producing ectoine microorganisms, ectoine may be hydroxylated into hydroxyectoine, also designated as 1,4,5,6-tetrahydro-2-methyl-5-hydroxy-4-pyrimidinecarboxylic acid, of number CAS 165542-15-4. This derivative of ectoine has been firstly identified in *Streptomyces parvulus* fermentation broth (Inbar & Lapidot, 1988). The enzyme ectoine hydroxylase (EctD) catalyzes the conversion of ectoine to hydroxyectoine through a regio- and stereo-specific hydroxylation reaction. This enzyme is found in many native ectoine producers.

Ectoine is secreted by the microorganisms. It may be recovered both from the culture medium, and from the producing microorganisms by cell lysis.

Optimization of ectoine extraction and purification from fermentation broth and/or naturally ectoine producing microorganisms has been investigated.

The patent CN112266362 discloses a method for extracting ectoine from *halophilic bacillus* bacteria, comprising: (i) crushing of freeze-dried bacteria, (ii) addition of a two-phase aqueous system based on ionic liquid comprising sulfate, (iii) recovering of the ionic liquid phase rich in ectoine, (iv) passing this ionic liquid phase on a microporous adsorption resin and then on a cation exchange resin, to remove impurities, (v) concentration of the ectoine contained in the eluate, and crystallization by vacuum drying, and a final step (vi) of redissolving the crude crystals in methanol, filtering and re-crystallizing ectoine to obtain high-purity ectoine crystals.

The international application WO 02/24664 describes a process of purification of ectoine from a fermentation broth comprising the following steps: (i) adsorption chromatography and elution, allowing to separate both compounds hydroxyectoine and ectoine since they have different adsorption propensities; (ii) fractional evaporation crystallization in methanol at room temperature and then at 55°C, wherein hydroxyectoine and ectoine have different solubilities; and (iii) repetition of steps (i) and (ii) a second time. The crystallized hydroxyectoine is filtered off. Eventually, pure ectoine is obtained. Fermentation broths are not specifically described; they might originate from microorganisms such as extremophilic bacteria and actinomycetes.

The article (Chen *et al.,* 2017) discloses a process for extraction and purification of ectoine from a fermentation broth of *Halomonas elongate,* comprising both ectoine and hydroxyectoine. After cell lysis, the processing operations are the following: (i) filtration, (ii) desalination, (iii) passing over a cation exchange resin, and then elution with a strong base, (iv) extraction and drying of "crude product", i.e., raw crystals, and (v) three successive steps of refining, including dissolution of the crude product into methanol at 60°C, filtration, concentration, and crystallization by cooling below 4°C. A composition comprising more than 98% of ectoine is obtained. Advantageously, the cell lysis is conducted at moderately high temperature (60-70°C) in presence of acid, which allows to remove the unfavorable odor and yellow color of the final product.

In these processes of the prior art, significant efforts are being made to separate ectoine from the by-product hydroxyectoine.

Genetic modifications of non-halophilic bacteria, such as *Corynebacterium glutamicum* or *Escherichia coli,* to express and produce ectoine, have been described (Schubert *et al.,* 2007, Becker *et al.,* 2013, He *et al.,* 2015, Ning *et al.,* 2016).

Ectoine purification processes have been implemented for recovering ectoine from these genetically modified microorganisms.

For example, the patent application CN 113637624A discloses a recombinant *Bacillus subtilis* strain producing ectoine. The genetic modifications consist of the expression of *EctA, EctB* and *EctC* genes originating from *Bacillus alcalophilus DTY1.* After culture, bacterial cells are broken and filtered through a membrane, and the ectoine present in the filtrate is adsorbed by cation exchange resin, and then eluted with ammonia.

The patent CN113528595B describes a genetically modified *Escherichia coli* strain, wherein the *ectABC* gene cluster derived from *Vibrio New Caledonia* has been introduced into the genome of said strain. After culture of the microorganisms, the fermentation broth is treated with a mix of chloroform and butanol to isolate ectoine into the water phase, which is then concentrated under reduced pressure. Steps of purification by polyamide adsorption and then elution with ethanol are performed, to collect an eluate that is concentrated and crystallized to obtain ectoine crystals.

While ectoine extraction and purification processes are known, there remains a need for cost-effective, simple, and rapid methods for obtaining compositions comprising high purity ectoine crystals with a minimum of by-products, and in particular compositions comprising high purity ectoine crystals without the by-product hydroxyectoine, or with only traces of it.

### Brief description of the invention

The present invention addresses the above needs, providing an improved process of extraction and purification of ectoine from a fermentation broth, which can be implemented on an industrial scale.

This process of extraction and purification of ectoine from a fermentation broth comprises the following successive steps:
a) Passing said fermentation broth through a cation exchange resin able to capture ectoine, and eluting ectoine, to obtain an ectoine aqueous solution;
b) Concentrating said ectoine aqueous solution and then drying it, to obtain raw crystals of ectoine;
c) Dissolving said raw crystals of ectoine into hot methanol;
d) Hot filtrating said methanol-ectoine mixture;
e) Crystallizing ectoine by cooling of the filtered methanol-ectoine mixture and recovering high purity ectoine crystals.

The present invention also provides a new composition comprising high purity ectoine crystals obtained by the process described above.

The present invention also provides a new composition comprising at least 98% in dry weight of high purity ectoine crystals, and less than 2% of by-products, wherein said by-products are chosen among the group consisting of: α-acetyl diaminobutyric acid (α-ADABA), γ-acetyl diaminobutyric acid (γ-ADABA), γ-aminobutyric acid (GABA) and any combination thereof.

### Detailed description of the invention

Before describing the present invention in detail, it is to be understood that the invention is not limited to particularly exemplified microorganism and/or methods and may, of course, vary. Indeed, various modifications, substitutions, omissions, and changes may be made without departing from the scope of the invention. It shall also be understood that the terminology used herein is for the purpose of describing particular embodiments of the invention only, and is not intended to be limiting.

All publications, patents and patent applications cited herein, whether supra or infra, are hereby incorporated by reference in their entirety. Furthermore, the practice of the present invention employs, unless otherwise indicated, conventional microbiological and molecular biological techniques that are within the skill of the art. Such techniques are well-known to the skilled person, and are fully explained in the literature.

Unless defined otherwise, all technical and scientific terms used herein have the same meanings as are commonly understood by one of ordinary skill in the art to which this invention belongs. Although any materials and methods similar or equivalent to those described herein can be used to practice or test the present invention, preferred material and methods are provided.

It must be noted that as used herein and in the appended claims, the singular forms "a," "an," and "the," include plural reference unless the context clearly dictates otherwise. Thus, for example, a reference to "a microorganism" includes a plurality of such microorganisms, and a reference to "an endogenous gene" is a reference to one or more endogenous genes, and so forth.

The terms "comprise," "contain," and "include" and variations thereof such as "comprising" are used herein in an inclusive sense, i.e., to specify the presence of the stated features but not to preclude the presence or addition of further features in various embodiments of the invention.

Genes and proteins are identified herein using the denominations of the corresponding genes in *E. coli* (e.g., *E. coli* K12 MG1655 having the Genbank accession number U00096.3) unless otherwise specified. However, in some cases use of these denominations has a more general meaning according to the invention and covers all of the corresponding genes and proteins in microorganisms.

A first aspect of the invention concerns a process for the extraction and purification of ectoine from a fermentation broth, comprising at least the successive steps of:
a) Passing said fermentation broth through a cation exchange resin able to capture ectoine, and eluting ectoine, to obtain an ectoine aqueous solution;
b) Concentrating said ectoine aqueous solution and then drying it, to obtain raw crystals of ectoine;
c) Dissolving said raw crystals of ectoine into hot methanol;
d) Hot filtrating said methanol-ectoine mixture;
e) Crystallizing ectoine by cooling of the filtered methanol-ectoine mixture, and recovering high purity ectoine crystals.

"Ectoine" is defined as the biochemical product of CAS number 96702-02-03. Ectoine is produced and secreted by microorganisms: it is recovered from the culture medium, after its secretion, and from the lysed microorganisms.

Ectoine production can be determined by standard analytical means known by the person skilled in the art. As a non-limiting example, ectoine may be quantified using HPLC or nuclear magnetic resonance, for example as described in Kuhlmann and Bremer, 2002 and Chen et al., 2017.

### Obtention and features of the fermentation broth

A "fermentation broth" designates, in the sense of the invention, both the culture medium where the microorganisms producing ectoine are cultivated, and the lysate obtained from the lysis of said microorganisms.

Usually, the fermentation broth is obtained by (i) lysis of the microorganisms into their culture medium, (ii) centrifugation to eliminate the cell debris, and (iii) collect of the supernatant which corresponds to the "fermentation broth containing ectoine".

The fermentation broth may also be pre-treated with any of the usual pre-treatment steps, well known by the person skilled in the art, for example addition of anti-foam agents, adjustment of the pH, and/or decantation.

According to a specific embodiment, said fermentation broth results from the culture of a genetically modified microorganism expressing heterologous genes encoding enzymes involved in ectoine synthesis, in a nutritive medium.

According to the invention, the terms "culture", "fermentative process", "fermentative production" or "fermentation" are used interchangeably to denote the growth of microorganism. This growth is generally conducted in fermenters with an appropriate growth medium adapted to the microorganism being used.

The terms "nutritive medium", "growth medium" and "culture medium" are used interchangeably to designate a medium (e.g., a sterile, liquid media) comprising nutrients essential or beneficial to the maintenance and/or growth of the cell such as carbon sources or carbon substrates, nitrogen sources; phosphorus sources, for example, monopotassium phosphate or dipotassium phosphate; trace elements (e.g., metal salts), for example magnesium salts, cobalt salts and/or manganese salts; as well as growth factors such as amino acids and vitamins. In particular, the inorganic culture medium can be of identical or similar composition to an M9 medium (Anderson, 1946), an M63 medium or a medium such as defined by Schaefer et al. (1999).

The term "source of carbon," "carbon source," or "carbon substrate" according to the present invention refers to any carbon source capable of being metabolized by a microorganism wherein the substrate contains at least one carbon atom. According to the present invention, said source of carbon is preferably at least one carbohydrate, and in some cases a mixture of at least two carbohydrates.

The term "source of nitrogen" according to the present invention refers to any nitrogen source capable of being used by the microorganism. Said source of nitrogen may be inorganic (e.g., (NH₄)₂SO₄) or organic (e.g., urea or glutamate). Preferably, said source of nitrogen is in the form of ammonium or ammonia solution.

The person skilled in the art is able to define the culture conditions for the microorganisms according to the invention.

The term "microorganism," as used herein, refers to a living microscopic organism, which may be a single cell or a multicellular organism and which can generally be found in nature. In the present context, the microorganism is preferably a bacterium, yeast or fungus.

Preferably, the microorganism is selected from the Enterobacteriaceae, Clostridiaceae, Bacillaceae, Streptomycetaceae, or Coηnebacteriaceae family or from among yeast, more preferably from the Saccharomycetaceae family.

Even more preferably, the microorganism is a species of *Escherichia, Klebsiella, Thermoanaerobacterium, Clostridium, Corynebacterium* or *Saccharomyces.*

Even more preferably, said Enterobacteriaceae bacterium is *Escherichia coli* or *Klebsiella pneumoniae,* said Clostridiaceae bacterium is *Clostridium acetobutylicum,* said Corynebacteriaceae bacterium is *Corynebacterium glutamicum,* or said Saccharomycetaceae yeast is *Saccharomyces cerevisiae.*

Most preferably, the genetically modified microorganism is of the species *Escherichia coli.*

The term "genetically modified microorganism" refers to a microorganism or a strain of microorganism that has been genetically modified or genetically engineered. This means, according to the usual meaning of these terms, that said modified microorganism is not found in nature and is genetically modified when compared to the "parental" microorganism from which it is derived. The "parental" microorganism may occur in nature (i.e., a wild-type microorganism) or may have been previously modified. The recombinant microorganisms of the invention may notably be modified by the introduction, deletion and/or modification of genetic elements. Such modifications can be performed, for example, by genetic engineering or by adaptation, wherein a microorganism is cultured in conditions that apply a specific stress on the microorganism and induce mutagenesis.

Said genetically modified microorganism expresses heterologous genes encoding enzymes involved in ectoine synthesis.

A microorganism may be genetically modified to express one or more exogenous or heterologous genes so as to overexpress the corresponding gene product (e.g., an enzyme, in the present case an enzyme involved in ectoine synthesis). The term "heterologous gene" indicates that a gene was introduced into a microorganism wherein said gene is not naturally occurring in said microorganism. The heterologous gene may be directly integrated into the chromosome of the microorganism, or be expressed extra-chromosomally within the microorganism by plasmids or vectors.

Heterologous genes encoding enzymes involved in ectoine synthesis are in particular:
- A gene encoding an enzyme having diaminobutyric acid acetyltransferase activity (EctA), preferably the gene *ectA* originating from *Halomonas elongate;*
- A gene encoding an enzyme having diaminobutyric acid aminotransferase activity (EctB), preferably the gene *ectB* originating from *Halomonas elongate; and*
- A gene encoding an enzyme having ectoine synthase activity (EctC), preferably the gene *ectC* originating from *Halomonas elongate.*

Advantageously, the microorganism does not comprise the *ectD* gene, encoding the enzyme involved in the conversion of ectoine to hydroxyectoine.

In a preferred embodiment of the invention, the fermentation broth results from the culture of a genetically modified microorganism which does not produce hydroxyectoine. Accordingly, the fermentation broth issued from the culture of this microorganism does not contain hydroxyectoine, or it contains less than 10 ppm of hydroxyectoine.

In addition of the expression of heterologous genes encoding enzymes involved in ectoine synthesis, the genetically modified microorganism may present further genetic modifications.

Genetic modification of non-halophilic bacteria, such as *Corynebacterium glutamicum* or *Escherichia coli,* to produce ectoine has been previously described (Becker et al., 2013, Schubert et al., 2007, He et al., 2015, Ning et al., 2016). In addition to expressing the *ectABC* genes for ectoine production, the following additional genetic modifications may be incorporated: deletion of *thrA* and *iclR,* and introduction of the heterologous feedback-resistant gene, *lysC.* Notably, the international application WO2021/156509 describes a genetically modified microorganism presenting an improved production of ectoine, which comprises the following modifications: expression of heterologous genes *ectA, ectB* and *ectC*; attenuation or deletion of *pykA* and *pykF* genes; and at least a 50% reduction in citrate synthase enzyme activity, as compared to an unmodified microorganism.

The genetically modified microorganism that is cultivated in the process of the invention may present at least one additional genetic modification, chosen among the group consisting of:
- attenuation or deletion of *pykA* gene, encoding a pyruvate kinase; attenuation or deletion of *pykF* gene, encoding a pyruvate kinase;
- any combination thereof.

In a specific embodiment of the invention, the genetically modified microorganism that is cultivated is further modified with the deletion of both endogenous genes *pykA* and *pykF.*

In a preferred embodiment, the genetically modified microorganism is from the species *Escherichia coli,* and comprises the following genetic modifications:
- expression of the heterologous gene *ectA* originating from *Halomonas elongate;*
- expression of the heterologous gene *ectB* originating from *Halomonas elongate;*
- expression of the heterologous gene *ectC* originating from *Halomonas elongate;*
- deletion of the endogenous genes *pykA* and *pykF.*

A fermentation broth resulting from the culture of this genetically modified *E. coli* strain contains between 5 and 10% of ectoine.

### Properties of the fermentation broth

The fermentation broth advantageously contains acidic amino acids.

Acidic amino acids are those with acidic side chains, specifically containing carboxylic acid groups with pKa measurements low enough to lose protons and become negatively charged. Acidic amino acids are also by their nature hydrophilic amino acids (meaning they like water, as opposed to hydrophobic amino acids), and polar amino acids (meaning they are positively charged, as opposed to nonpolar amino acids).

Acidic amino acids are aspartic acid and glutamic acid.

These acidic amino acids are produced during the culture of the microorganisms, or are added after the step of culture, to improve recovery of ectoine during elution step.

Indeed, the presence of acidic amino acids, which are decarboxylated into by-products, improves the recovery of ectoine during the elution of step a).

In another embodiment, the fermentation broth contains at least one derivative of said acidic amino acids, such as γ-aminobutyric acid (GABA, a decarboxylated form of glutamate),

### Process of extraction and purification of ectoine

The process according to the invention comprises at least the successive steps of:
a) Passing said fermentation broth through a cation exchange resin able to capture ectoine, and eluting ectoine, to obtain an ectoine aqueous solution;
b) Concentrating said ectoine aqueous solution and then drying it, to obtain raw crystals of ectoine;
c) Dissolving said raw crystals of ectoine into hot methanol;
d) Hot filtrating said methanol-ectoine mixture; and
e) Crystallizing ectoine by cooling of the filtered methanol-ectoine mixture, and recovering high purity ectoine crystals.

In a specific embodiment of the invention, a preliminary step of clarification of the fermentation broth is carried out before step (a).

A step of clarification designates a step of removing the insoluble organic impurities from said fermentation medium. The clarification of the medium is carried out by any method known as such by those skilled in the art, which method is chosen, for example, from the group consisting of heating, flocculation, decanting, membrane techniques (microfiltration, ultrafiltration, diafiltration, nano-filtration and reverse osmosis) and centrifugation.

The clarified fermentation broth does not contain hydroxyectoine, or contains less than 10 ppm of hydroxyectoine, as measured for example with a Waters Acquity UPLC coupled with an AB Sciex API3200 mass spectrometer fitted with an electrospray source.

In a specific embodiment, the clarified fermentation broth presents the following features:
- it contains at least one by-product, in particular chosen among the group consisting of: α-acetyl diaminobutyric acid (α-ADABA), γ-acetyl diaminobutyric acid (γ-ADABA), GABA, and any combination thereof;
- optionally, it contains alanine, and.
- it contains less than 10 ppm of hydroxyectoine.

Step (a) uses a cation exchange resin able to capture ectoine (captation step), which is preferentially a strong cationic resin such as bead-like products which have a sulfonic acid group in a cross-linked styrene frame. The person skilled in the art knows, for example, the commercially available exchange resins designated as POROS^{™} XS from ThermoFisher and DIAION^{™} PK or LK from Mitsubishi chemical.

The elution of ectoine is achieved by following the instructions of the distributor of the exchange resin. It is generally obtained by addition to the cation exchange resin of a strong base such as, for example, a sodium hydroxide (NaOH) solution or ammonium hydroxide solution (NH₄OH). The eluate is controlled at the column outlet: once ammonium ions are eluted, the elution step is over.

Interestingly, it has been noted that recovery of ectoine by elution with a strong base is improved in presence of the amino acid derivative GABA.

Step (b) corresponds to the concentration of ectoine into the aqueous solution containing it: this concentration is achieved by any mean known by the person skilled in the art, such as evaporation of the aqueous solution. The step also comprises the drying of the solution, i.e., complete elimination of water. The most usual mean for drying is the heating of the solution until all water is evaporated, but it can be performed by any other mean known by the person skilled in the art, such as vacuum drying.

At the end of step (b), raw crystals are obtained: they contain at least 70%, 75%, or 80% of ectoine; preferentially, raw crystals contain about 85% of ectoine and about 15% of by-products and/or residual water.

In a specific embodiment of the invention, the obtained raw crystals of ectoine comprise less than10% of residual water, and preferentially less than 5%.

Step (c) corresponds to the dissolution of raw crystals of ectoine obtained in step (b) into hot methanol. This step has been primarily disclosed in (Chen et al., 2017): it was intended to separate ectoine from hydroxyectoine. Surprisingly, the inventors of the present application have discovered that this step is also useful for purifying raw ectoine crystals which do not comprise hydroxyectoine.

Dissolution of the raw ectoine crystals into hot methanol is performed to obtain a slurry mixture that is heated at a temperature comprised between 45°C and 65°C, preferentially at a temperature comprised between 50°C and 60°C, and more preferentially at a temperature of about 55°C. The term "slurry" designates a mixture of denser solids suspended in liquid, usually water.

This dissolution is preferentially performed under agitation.

Step (d) corresponds to a step of hot filtration of said methanol-ectoine mixture. The term "hot" designates a temperature comprised between 45°C and 65°C, preferentially at a temperature comprised between 50°C and 60°C, and more preferentially at a temperature of about 55°C.

This filtration is performed according to any mean known by the person skilled in the art, for example by passing the methanol-ectoine mixture over a microfilter.

In a preferred embodiment, the hot filtration is performed with a microfilter having pore diameter comprised between 0.5 and 25 µm, preferably between 1 and 15 µm.

Optionally, the hot filtration is performed in presence of a filter aid such as activated carbon, diatomaceous earth or perlite.

The person skilled in the art will adapt this filtration step according to his general knowledge. The goal of this step of filtration is removing solid and/or insoluble impurities present in the methanol-ectoine mixture, which are not dissolved into the methanol and therefore will be retained by the microfilter. The removing solid and/or insoluble impurities are designated by the term "insoluble cake".

For example, by-products such as α-ADABA, γ-ADABA and alanine can be under a solid form into the hot methanol mixture. The optional filter aid can also be removed with this step of hot filtration.

Step (e) is a step of recrystallization of ectoine; this step is performed by cooling the filtered methanol-ectoine mixture, to a temperature comprised between -15°C and 10°C.

The obtained ectoine crystals are then separated by spinning or filtration, and residual methanol is evaporated by vacuum drying.

The obtained crystals are designated as "high purity ectoine crystals" since they contain at least 98% in dry weight of ectoine crystals, and less than 2% of by-products.

The liquid methanol remaining after recovery of the ectoine crystals still contains some dissolved ectoine together with impurities. It may be partially recycled to step (c) in order to increase ectoine recovery yield. The ratio of recycled methanol over fresh methanol used in step (c) is to be adjusted in order to keep ectoine crystals to the expected purity. The purged methanol (fraction that is not recycled) may be treated by distillation ultimately, in order to be reused as fresh methanol.

### Compositions of high purity ectoine crystals

The present invention also concerns a composition comprising high purity ectoine crystals obtained by the process as described above.

The present invention also concerns a composition comprising at least 98% in dry weight of high purity ectoine crystals, and less than 2% of by-products, wherein said by-products are chosen among the group consisting of: α-acetyl diaminobutyric acid (α-ADABA), γ-acetyl diaminobutyric acid (γ-ADABA), γ-aminobutyric acid (GABA) and any combination thereof.

In a specific embodiment of the invention, said composition further comprises alanine as a by-product.

Advantageously, the composition comprising high purity ectoine crystals does not comprise hydroxyectoine.

The present invention also concerns a composition comprising high purity ectoine crystals obtained by the process as described above, presenting the following features:
- comprising at least 98% in dry weight of high purity ectoine crystals;
- comprising less than 2% of by-products, wherein said by-products are chosen among the group consisting of: α-acetyl diaminobutyric acid (α-ADABA), γ-acetyl diaminobutyric acid (y-ADABA),y-aminobutyric acid (GABA) and any combination thereof;
- optionally, further comprising alanine as a by-product; and
- comprising no hydroxyectoine.

### Examples

The present invention is further defined in the following examples. It should be understood that these examples, while indicating preferred embodiments of the invention, are given by way of illustration only. The person skilled in the art will readily understand that these examples are not limitative and that various modifications, substitutions, omissions, and changes may be made without departing from the scope of the invention.

### Example 1: Recovery of an ectoine solution from fermentation broth of Escherichia coli strain using a cation exchange resin (step a).

### 1.1 Fermentation, pre-treatment and clarification steps

*Escherichia coli* EC0044F04 recombinant strain that is cultivated in the process of the invention is genetically modified for expression of the following heterologous genes encoding enzymes involved in ectoine synthesis:
- A gene encoding an enzyme having diaminobutyric acid acetyltransferase activity (EctA), preferably the gene *ectA* originating from *Halomonas elongate;*
- A gene encoding an enzyme having diaminobutyric acid aminotransferase activity (EctB), preferably the gene *ectB* originating from *Halomonas elongate; and*
- A gene encoding an enzyme having ectoine synthase activity (EctC), preferably the gene *ectC* originating from *Halomonas elongate.*

*Escherichia coli* EC0044F04 recombinant strain is further genetically modified with the deletion of endogenous genes *pykA* and *pykF.*

*Escherichia coli* EC0044F04 recombinant strain was fermented in 20L fed-batch fermenter to produce ectoine.

Fermentation was performed on minimum medium with a feed of carbohydrates (such as glucose) as carbon source. The temperature of the culture was maintained constant at 37°C and the pH was permanently adjusted to values between 6,0 and 7,5, preferentially 6,8 using an NH₄OH 28% solution.

Fermentation is usually stopped when ectoine titer reaches a proportional value comprised between 6% and 12% in the broth. The broth is then recovered to perform desactivation steps such as acidification, using a strong mineral acid, and thermal treatment.

Pre-treatment of the broth has an impact on its composition, especially for the presence of by-products such as N-acetyl-diamino-butyric acid (ADABA,y and α), glutamate and γ-amino butyric acid (GABA, a decarboxylated form of glutamate).

**Table 1: Evolution of the composition of the fermentation broth after pre-treatment steps.**

| **Sample reference** | | **FfEC_00214** | **EC22230HB T0** | **EC22230HB TF** | **Specific Mass Balance after acidification** | **Specific Mass Balance after thermal treatment** |
|---|---|---|---|---|---|---|
| | | | | | | |
| **Description** | | **Fermentation broth** | **Acidified broth** | **Desactivated broth** | | |
| **Compound** | **unit** | | | | | |
| **Ectoine** | g/kg | 68,9 | 64,6 | 67,3 | 97% | 101% |
| | kg | 1,4 | 1,3 | 1,3 | | |
| **α-ADABA** | mM | 0,0 | 0,0 | 16,9 | no α-ADABA at the end of fermentation; mass balance with both forms of ADABA => | 101% |
| | g | 0 | 0 | 53 | | |
| **γ-ADABA** | mM | 32,1 | 29,3 | 13,8 | 95% | 45% |
| | g | 101 | 96 | 43 | | |
| **Alanine** | mM | 0,0 | 1,2 | 1,0 | alanine not quantified at the end of fermentation | 78% |
| | g | 0 | 2 | 2 | | |
| **GABA** | mM | 0,0 | 44,4 | 43,8 | no GABA at the end of fermentation | 95% |
| | g | 0 | 93 | 89 | | |
| **Glutamate** | mM | 28,5 | 0,4 | 0,7 | 1% | transformed into GABA |
| | 9 | 82 | 1 | 2 | | |

Ectoine titer and relative purity are lowered after acidification, due to the increase of inorganic anions content. Relative purity of ectoine in broth remains stable after thermal treatment, however the titer is lowered due to apparition of condensates in fermentation broth. Pre-treatment causes the total transformation of glutamate into GABA by decarboxylation, and partial transformation of γ-ADABA into α-ADABA by transacetylation.

After pre-treatment of the fermentation broth, cells and precipitated proteins are separated using clarification steps. Centrifugation, microfiltration and ultrafiltration are used for clarification of the fermentation broth. Biomass elimination has an impact on the relative purity of ectoine.

Hydroxyectoine identification and quantification are performed with a Waters Acquity UPLC coupled with an AB Sciex API3200 mass spectrometer fitted with an electrospray source. The MS is operated in the positive ion and multiple-reaction monitoring (MRM) mode. The chromatographic separation is achieved on Acquity UPLC -HSS T3 C18 column at 40°C using an isocratic elution program at a flow rate of 0.4 mL/min; mobile phase is acetonitrile/water (1:99 v/v) (containing 0.1% formic acid). Hydroxyectoine is identified by its retention time and characteristic ions, and is quantified using authentic standard.

Results show that the clarified fermentation broth contains less than 10 ppm of hydroxyectoine.

The table 2 below presents the mass balance of a clarification step performed on a centrifuge decantor Lemitec MD60. Biomass elimination increases the relative purity of ectoine versus total dry matter content.

**Table 2: Results of clarification step EC23279DC.**

| | **Desactivated broth** | **Supernatant** | **Sludges** | **Mass balance** | **Ectoine Yield** |
|---|---|---|---|---|---|
| **Mass** | 18,9 | 16,4 | 1,7 | 96,6% | |
| **[Ectoine] (g/kg)** | 64,5 | 67,2 | 51,9 | 97,4% | 90,1% |
| **Ectoine mass (kg)** | 1,2 | 1,1 | 0,1 | | |
| **[Dry matter content] %** | 12,2% | 11,4% | 31,9% | 104,4% | |
| **Dry matter mass (kg)** | 2,3 | 1,9 | 0,6 | | |
| **Relative purity of ectoine vs dry matter (%)** | 53% | 59% | 16% | | |

### 1.2 Cation exchange resin and elution steps (step a)

The clarified fermentation broth is then passed through a cation exchange resin in order to bind the cations. The volume of the clarified fermentation broth has to be calculated regarding to resin capacity, so that saturation of the resin bed is not overpassed. Once the feeding solution has passed through the resin bed, water is fed to rinse the resin bed before the elution step. The elution is performed using a diluted sodium hydroxide solution or ammonium hydroxide solution. The table 3 below presents the results of a cation exchange resin trial performed on a 300mL glass column filled by 200mL of Dupont FPC11Na resin.

**Table 3: Results of trial EC23263RE: captation before elution of ectoine with ammonia solution.**

| **Description** | **Feed** | **Eluate** | **Global mass balance** | **Yield in eluate** |
|---|---|---|---|---|
| **Mass** | 340,6 | 327,3 | 95,3% | |
| **[Ectoine] (g/kg)** | 80,3 | 73,9 | 98,2% | 88,4% |
| **Ectoine mass (g)** | 27,3 | 24,2 | | |
| **[γ-ADABA] (g/kg)** | 2,9 | 2,35 | | 78,7% |
| **γ-ADABA mass (mg)** | 976,6 | 768,3 | | |
| **[α-ADABA] (g/kg)** | 4,7 | 4,12 | | 83,4% |
| **α-ADABA mass (mg)** | 1616,3 | 1348,8 | | |
| **[GABA] (g/kg)** | 1,7 | 1,55 | | 88,6% |
| **GABA mass (mg)** | 572,3 | 506,94 | | |
| **Dry matter content %** | 12,1% | 8,2% | | |
| **Relative purity of ectoine vs dry matter (%)** | 67% | 90,6% | | |

This process step increases the relative purity of ectoine versus total dry matter content. This is especially due to organic and inorganic anions elimination. However, the relative content of ADABA or GABA is almost the same in alimentation and resin step eluate.

### Example 2: Recovery of raw ectoine powder from ectoine solution (step b)

Eluted ectoine solution is concentrated before a drying step. Concentration of ectoine solution has been performed using a Buchi Rotavapor in order to recover a concentrated solution of ectoine. Several concentration runs are performed, from ectoine solutions of various purity vs dry matter content.

**Table 4: Example of a concentration step (EC23275RV): concentration of ectoine solution from EC23263RE resin trial.**

| **Flux** | **Feed** | **Concentrate** | **Mass balance** |
|---|---|---|---|
| **Mass (g)** | 327,3 | 67,8 | 98% |
| **[Ectoine] (g/kg)** | 73,9 | 353,0 | 103% |
| **Ectoine mass (g)** | 24,2 | 23,9 | |
| **Dry matter (% w/w)** | 8,2% | 39,4% | 99% |
| **Ectoin purity vs dry matter (%)** | 90,6% | 89,6% | |

Ectoine purity remains almost stable after concentration by evaporation of water in rotavapor. The concentrate of ectoine is then dried using a Buchi atomizer spray dryer B290. A powder containing ectoine is recovered after drying. Several runs of drying are performed, and a pool of the recovered powders is done.

**Table 5: Composition of raw ectoine powder obtained from different concentrates (pool of dry product of atomisation trials referenced EC23039AT, EC23040AT, EC23041AT, EC23044AT and EC23047AT). LOQ: Limit of quantification.**

| **Molecule** | **EC23048MX** |
|---|---|
| **ECTOINE** | 86,1% |
| **ALANINE** | <LOQ |
| **α-ADABA** | 3,4% |
| **GABA** | 1,0% |
| **γ-ADABA** | 3,4% |

Relative humidity of this pool of powder is measured before recrystallisation steps. Value is 1,3% w/w of water.

Relative purity of ectoine of this pool is lower than the one presented in table 1 of example 2, due to higher content of GABA, α-ADABA and γ-ADABA in the fermentation broths used to obtain this powder, regarding the fermentation broth used for EC23263RE trial.

Alanine is mostly co-eluted with γ-ADABA by HPLC, so it is not possible to quantify this compound in this dry product, especially due to much higher content of γ-ADABA regarding residual alanine.

### Example 3: Recovery of high purity ectoine powder from raw ectoine powder by recrystallization in methanol (steps c to e)

Raw ectoine powder is mixed in methanol, and the slurry obtained is heated from 45 to 55°C. The amount of powder added into methanol is calculated so that ectoine content is comprised between 9 and 12% w/w. After one hour at the heating temperature target, almost all ectoine is dissolved, however the slurry still contains solid particles. Separation of these impurities was performed using hot filtration (double jacketed filter cell from BHS). In order to enhance filtration performances, filter has been added before heating of the slurry.

**Table 6: 5 different operating conditions for the dissolution of ectoine in methanol and hot filtration of raw ectoine slurry in methanol.**

| **Trial reference** | 1 | 2 | 3 | 4 | 5 |
|---|---|---|---|---|---|
| **Heating Temperature (°C)** | 55 | 45 | 55 | 55 | 55 |
| **[Ectoine] in slurry (%wt)** | 10,64% | 8,86% | 10,58% | 8,81% | 10,27% |
| **Pore size of hot filtration filter** | 5µm | 5µm | 1,2µm | 11µm | 11 µm |
| **Filter aid** | No | No | No | 6% vs raw powder | 6% vs raw powder |
| **Filtration pressure (barg)** | 1 to 2,5 | 1 | 0,5 to 1,5 | 1 to 2 | 1 to 2 |

**Table 7: Composition of insoluble cake from hot filtration. LOQ: Limit of quantification.**

| | **EC23048MX** | **1** | **2** | **3** | **4** | **5** |
|---|---|---|---|---|---|---|
| **Ectoine (%)** | 86,1% | 12,5% | 32,3% | 28,7% | 10,0% | 11,3% |
| **ALANINE (%)** | <LOQ | <LOQ | <LOQ | <LOQ | <LOQ | <LOQ |
| **α-ADABA (%)** | 3,4% | 1,1% | 0,4% | 1,9% | 0,7% | 0,4% |
| **GABA (%)** | 1,0% | 0,1% | 0,1% | 0,3% | 0,0% | 0,0% |
| **γ-ADABA (%)** | 3,4% | 31,5% | 4,5% | 17,5% | 5,8% | 3,9% |

Hot filtration separates insoluble by-products from the slurry, especially γ-ADABA.

Recovered filtrate is then cooled, so that ectoine crystallizes. Temperature is decreased to a temperature between 10°C and -10°C. After one hour at the target temperature, the slurry is filtered on a 11µm filter to recover the pure ectoine crystals from the methanol solution.

**Table 8: Composition comprising high purity ectoine crystals recovered after recrystallization step. LDQ: Limit of quantification.**

| **Dry product reference** | **EC23048MX** | **1** | **2** | **2** | **4** | **5** |
|---|---|---|---|---|---|---|
| Ectoine (%) | 86,1% | 99,3% | 99,2% | 99,2% | 99,6% | 99,6% |
| **ALANINE** (%) | <LOQ | <LOQ | <LOQ | <LOQ | <LOQ | <LOQ |
| α-ADABA (%) | 3,4% | 0,04% | 0,04% | 0,07% | 0,05% | 0,06% |
| GABA (%) | 1,0% | 0,01% | 0,01% | 0,02% | 0,02% | 0,02% |
| γ-ADABA (%) | 3,4% | 0,05% | 0,14% | 0,35% | 0,05% | 0,08% |

Purity of recrystallized ectoine in methanol is higher than 99% in the five tested conditions.

Application of the different steps of the process of the invention allows to recover high purity ectoine crystals from a fermentation broth in which relative purity of ectoine versus dry matter content is comprised between 50% and 70%.

### REFERENCES

### PATENTS

JPH0331265
WO 2010/006792
CN112266362
WO 02/24664
CN113637624
CN113528595
WO 2021/156509

### SCIENTIFIC LITTERATURE

∘ Anderson, (1946), Proc. Natl. Acad. Sci. USA., 32:120-128.
∘ Becker et al., (2013), Microbial Cell Factories. 12(1):110
∘ Chen et al, (2017). World J Microbiol Biotechnol. Jun;33(6):116.
∘ Fallet et al., (2010), Biotechnology and Bioengineering. 107(1):124-33. ∘ Goraj et al., (2019), Advancements of Microbiology, 58(3): 339-349.
∘ He et al., (2015) Microb Cell Fact. 14(1), 55.
∘ Inbar L, Lapidot A. (1988) J Biol Chem. Nov 5;263(31):16014-22
∘ Kuhlmann and Bremer, (2002), Appl. Environ. Microbiol., 68:772-783
∘ Ning et al., (2016), Metab Eng. 36:10-18.
∘ Schubert et al., (2007) Appl Environ Microbiol. 73(10): 3343-3347.
∘ Schaefer et al., (1999), Anal. Biochem. 270: 88-96.
∘ Strong et al., (2016), Bioresour Technol., 215:314-323

## Claims

1. Process for the extraction and purification of ectoine from a fermentation broth, comprising at least the successive steps of:
a) Passing said fermentation broth through a cation exchange resin able to capture ectoine, and eluting ectoine, to obtain an ectoine aqueous solution;
b) Concentrating said ectoine aqueous solution and then drying it, to obtain raw crystals of ectoine;
c) Dissolving said raw crystals of ectoine into hot methanol;
d) Hot filtrating said methanol-ectoine mixture;
e) Crystallizing ectoine by cooling of the filtered methanol-ectoine mixture and recovering high purity ectoine crystals.

2. Process for the extraction and purification of ectoine from a fermentation broth according to claim 1, wherein said fermentation broth results from the culture of a genetically modified microorganism expressing heterologous genes encoding enzymes involved in ectoine synthesis, in a nutritive medium.

3. Process for the extraction and purification of ectoine from a fermentation broth according to claim 2, wherein said genetically modified microorganism is of the species *Escherichia coli.*

4. Process for the extraction and purification of ectoine from a fermentation broth according to claim 2 or 3, wherein said genetically modified microorganism is further modified with the deletion of endogenous genes *pykA* and *pykF.*

5. Process for the extraction and purification of ectoine from a fermentation broth according to anyone of claims 2 to 4, wherein said fermentation broth comprises acidic amino acids, and wherein said acidic amino acids are produced during said culture of a genetically modified microorganism or added after.

6. Process for the extraction and purification of ectoine from a fermentation broth according to anyone of claims 1 to 5, further comprising a step of clarification of the fermentation broth, carried out before step (a).

7. Process for the extraction and purification of ectoine from a fermentation broth according to claim 6, wherein the clarified fermentation broth:
- contains at least one by-product chosen among the group consisting of: α-acetyl diaminobutyric acid (α-ADABA), γ-acetyl diaminobutyric acid (γ-ADABA), γ-aminobutyric acid (GABA), and any combination thereof,
- optionally contains alanine, and
- contains less than 10 ppm of hydroxyectoine.

8. Process for the extraction and purification of ectoine from a fermentation broth according to anyone of claims 1 to 7, wherein in step (b), the obtained raw crystals of ectoine comprise less than10% of residual water, and preferentially less than 5%.

9. Process for the extraction and purification of ectoine from a fermentation broth according to anyone of claims 1 to 8, wherein in step (c), the dissolution into hot methanol is performed at a temperature comprised between 45°C and 65°C, preferentially at a temperature comprised between 50°C and 60°C, and more preferentially at a temperature of 55°C.

10. Process for the extraction and purification of ectoine from a fermentation broth according to anyone of claims 1 to 9, wherein in step (d), the hot filtration is performed with a microfilter having pore diameter comprised between 0.5 and 25 µm, preferably between 1 and 15 µm, optionally in presence of a filter aid.

11. Process for the extraction and purification of ectoine from a fermentation broth according to anyone of claims 1 to 10, wherein in step (e), the chilled filtered methanol-ectoine mixture reaches a temperature comprised between -15°C and 10°C.

12. Composition comprising high purity ectoine crystals obtained by the process according to anyone of claims 1 to 11.

13. Composition comprising at least 98% in dry weight of high purity ectoine crystals, and less than 2% of by-products, wherein said by-products are chosen among the group consisting of: α-acetyl diaminobutyric acid (α-ADABA), γ-acetyl diaminobutyric acid (y-ADABA),y-aminobutyric acid (GABA) and any combination thereof.

14. Composition according to claim 13, wherein said composition further comprises alanine as a by-product.

15. Composition according to claim 13 or 14, wherein said composition does not comprise hydroxyectoine.
